Europäisches Patentamt

European Patent Office

Office européen des brevets

(19)

(11) Veröffentlichungsnummer: **0 166 946 B1**

# EUROPÄISCHE PATENTSCHRIFT

(12)

(45) Veröffentlichungstag der Patentschrift: **24.07.91**

(21) Anmeldenummer: **85106375.0**

(22) Anmeldetag: **06.05.82**

Die Akte enthält technische Angaben, die nach dem Eingang der Anmeldung eingereicht wurden und die nicht in dieser Patentschrift enthalten sind.

(51) Int. Cl.5: **A61K 7/00, A61K 9/10**

(60) Veröffentlichungsnummer der früheren Anmeldung nach Art. 76 EPÜ: **0 065 929**

(54) **Kosmetisches Mittel.**

(30) Priorität: **08.05.81 AT 2071/81**

(43) Veröffentlichungstag der Anmeldung:
**08.01.86 Patentblatt 86/02**

(45) Bekanntmachung des Hinweises auf die Patenterteilung:
**24.07.91 Patentblatt 91/30**

(84) Benannte Vertragsstaaten:
**AT BE CH DE FR GB IT LI LU NL SE**

(56) Entgegenhaltungen:
**FR-A- 1 603 602**
**FR-A- 2 326 914**

**DERWENT JAPANESE PATENTS REPORT, Nr. 47, 15. Januar 1971, Artikel Nr. 87444R, Derwent Publication Ltd., GB; & JP - A - 70 37 292**

**Dr. OTTO-ALBRECHT NEUMÜLLER: "Römpps Chemie-Lexikon", 7. Auflage, 1973, Seiten 1009-1010, Franckh'sche Verlagshandlung, Stuttgart, DE.**

(73) Patentinhaber: **SCHERING WIEN Ges.m.b.H.**
**Scheringgasse 2**
**A-1140 Wien(AT)**

(72) Erfinder: **Stindl, Wolfgang**
**Kleinhöfleiner Hauptstrasse 24**
**A-7000 Eisenstadt(AT)**

(74) Vertreter: **Pfeifer, Otto, Dipl.-Ing. et al Patentanwälte, Dipl.-Ing. Dr. techn. Schütz, Alfred, Dipl.-Ing. Holzer, Walter Dipl.-Ing. Pfeifer, Otto Fleischmanngasse 9**
**A-1040 Wien(AT)**

## Beschreibung

Die Erfindung betrifft, wie untenstehend im einzelnen gekennzeichnet, ein kosmetisches Mittel in Form einer Salbe, Paste oder Creme, das lipophilen Wirkstoff, eine Fettphase, eine wässerige Phase, Emulgatoren, Konservierungsmittel sowie Duftstoffe enthält.

Es sind zahlreiche derartige kosmetische Cremes oder Salben, insbesondere sogenannte Nähr- und Feuchtigkeitscremes bekannt, die entweder auf einer Öl/Wasser-Emulsion oder einer Wasser/Öl-Emulsion basieren. Das mit solchen Cremes verbundene Problem liegt allerdings darin, daß die einen unterschiedlichen Fett- und/oder Feuchtigkeitsbedarf aufweisenden Bereiche der Haut bzw. verschiedene Hauttypen beim Auftragen solcher Cremes nicht ihrem speziellen Bedarf entsprechend versorgt werden können. Gemessen am Fett- und/oder Feuchtigkeitsbedarf der Haut wird beim Auftragen solcher Cremes einzelnen Hautbereichen gewöhnlich entweder zu viel oder zu wenig Fettphase, Nährstoffe oder Feuchtigkeit zugeführt. Auch ein nacheinander erfolgendes Auftragen von Cremes auf Basis des einen und dann des anderen Emulsionstyps bringt hier keine Abhilfe.

Aufgabe der Erfindung ist nun die Schaffung von kosmetischen Salben, Paten oder Cremes, mit deren Hilfe unterschiedliche Hautbereiche gleichzeitig entsprechend ihrem spezifichen Fett-, Nahrstoff- und/oder Feuchtigkeitsbedarf versorgt werden können.

Die Lösung dieser Aufgabe erfolgt gemäß der Erfingung durch Schaffung eines speziellen Emulsionstypus, nämlich einer feinstdispersen Mischung zweier Emulsionskomponenten, wobei die Teilchengröße jeder der beiden, die feinstdisperse Mischung bildenden Emulsionskomponenten (die jede für sich eine Emulsion darstellt) 2 bis 50 $\mu$m beträgt. Dieser spezielle Typus von Emuslionen soll nachstehend kurz mit "Doppelemulsion" bezeichnet werden, wobei bemerkt wird, daß in der älteren Literatur gelegentlich der Ausdruck "Doppelemulsion" - aufgrund einer irrtümlichen Interpretation des unter einem einfachen Lichtmikroskop entstehenden Eindruckes - auch für Mischemulsionen, d.s. solche mit zwei kontinuierlichen Phasen ohne diskontinuierliche Phase, gebraucht worden ist. Solche bekannten Mischemulsionen werden im Rahmen der Erfindung vom Ausdruck "Doppelemulsion" ebensowenig umfaßt wie Sekundär- ("Zweifach"-) Emulsionen.

In der FR-A-1,603.602 wird eine Salbe, Creme o.ä. beschrieben, welche eine verzögerte Abgabe eines in ihr enthaltenen Wirkstoffes bewirkt. Wie aus Seite 1, Zeile 17 ff. hervorgeht, handelt es sich um einen pharmazeutischen Wirkstoffträger, der im einfachsten Fall eine Wasser-in-Öl-in-Wasser-Emulsion (W/O/W) ist (siehe Seite 1, Zeilen 25 bis 29). Der Ausdruck "emulsion double" bedeutet in der Sache nicht eine "Doppelemulsion" im Sinne der oben gegebenen Definition, sondern eine Sekundäremulsion, wie sich auch aus der Herstellungsanweisung auf Seite 32 und 33 im Punkt 17 ff. - speziell 8 bis 20 - ergibt. Aus diesen Angaben in der Veröffentlichung ist ersichtlich, daß eine erhaltene flüssige Emulsion in eine wässerige Tensidlösung eingearbeitet wird, wobei, wie sich aus dem Vorliegen nur einer eine Emulsion darstellenden Ausgangskomponente ergibt, keine Doppelemulsion entstehen kann.

Die Salbe, Creme o.ä. der FR-A-2,326.914 hat die Form einer Wasser/Öl/ Wasser-Emulsion. Wie aus Seite 2, Zeilen 14 bis 17 dieser Veröffentlichung zu ersehen ist, ist die komplexe Emulsion gemäß dieser Schrift eine solche vom Typus Wasser/Öl/Wasser, also von einer Doppelemulsion verschieden und mit dieser nicht vergleichbar. Soweit in dieser Veröffentlichung von verschiedenen Emulsionstypen W/O und O/W die Rede ist, so werden diese Typen fakultativ mit den Zielen eingesetzt, die komplexe Emulsion vom Wasser/Öl/Wasser-Typus zu bilden.

In der JA-A-7 037 292 wird eine sehr stabile wässerige Aerosollösung die somit ein treibmittelhaltiges, flüssiges Produkt und kein halbfestes, kosmetisches Produkt darstellt, beschrieben. Die in der genannten JA-Patentveröffentlichung angegebene Verfahrensweise zur Herstellung der Aerosollösung führt außerdem nicht zu einer Doppelemulsion. Alle diese bekannten Mittel eignen sich nicht zur Lösung der erfindungsgemäßen Aufgabe.

Die erfindungsgemäße Aufgabe wird durch die Bereitstellung eines kosmetischen Mittels in Form einer Salbe, Paste oder Creme, das lipophilen Wirkstoff, eine Fettphase, eine wässerige Phase, Emulgatoren, Konservierungsmittel sowie Duftstoffe enthält, gelöst, das dadurch gekennzeichnet ist, daß es den lipophilen Wirkstoff, die Fettphase und wässerige Phase in Form einer feinstdispersen Mischung einer einen Öl/Wasser-Emulgator und Konservierungsmittel enthaltenden Öl/Wasser-Emulsion und einer einen Wasser/Öl-Emulgator enthaltenden Wasser/Öl-Emulsion mit einer Teilchengröße von jeweils 2 bis 50 $\mu$m enthält, wobei der Wirkstoff Pur-oba-Öl ist.

Mit Hilfe der die feinstdisperse Mischung der beiden Emulsionstypen enthaltenden Creme kann auf der Haut je nach ihrer Feuchtigkeit auf ihren unterschiedlichen Bereichen bzw. je nach Hauttypus ein entsprechender Öl/Wasser- oder Wasser/Öl-Hydrolipidfilm erzeugt werden.

Das erfindungsgemäße Mittel kann nach einem Verfahren hergestellt werden, das dadurch gekennzeich-

net ist, daß man aus lipophilem Wirkstoff, Fettphase, wässeriger Phase, Öl/Wasser-Emulgator und Konservierungsmittel eine Öl/Wasser-Emulsion und aus Fettphase, wässeriger Phase und Wasser/Öl-Emulgator eine Wasser/Öl-Emulsion herstellt und däß man die beiden Emulsionen unter Vakuum bei einer Temperatur zwischen 20 und 40° C innig verrührt und darauf die erhaltene feindisperse Mischung mit Duftstoffen versetzt.

Wenn es auch anscheinend im Hinblick auf die unterschiedlichen Bedürfnisse der Haut als naheliegend angesehen werden könnte, einfach die beiden Emulsionstypen zur Herstellung einer Creme zu vermischen, um so den oben geschilderten Bedürfnissen der Haut zu genügen, so ist diese Vorgangsweise nicht ohne weiteres möglich. Dies deswegen, weil die beiden Emulsionstypen bisher nicht stabil nebeneinander aufrechterhalten werden konnten, sondern vielmehr in Abhängigkeit von den verwendeten Bestandteilen zumindest bald nach der Verarbeitung eine Umwandlung des einen in den anderen Emulsionstyp auftritt, so daß im Endeffekt nur ein Emulsionstyp in der hergestellten Creme erhalten bleibt.

Wenn nun die beiden Emulsionstypen bei Temperaturen zwischen 20 und 40° C, vorzugsweise bei 30° C, unter Vakuum besonders schonend, dessen ungeachtet jedoch sehr intensiv, in einem Rührwerksbehälter vereinigt werden, so daß schließlich eine sehr feine Dispersion der beiden Emulsionen, deren Teilchengröße zwischen 2 und 50 $\mu$m, vorzugsweise zwischen 5 und 15 $\mu$m beträgt, erzielt wird, dann bleiben die beiden Emulsionstypen unverändert nebeneinander in der hergestellten Creme erhalten. Dieses Emulsionssystem bleibt bei erheblicher Verdünnung, z.B. mit Flüssigphase, stabil. Es wird angenommen, daß für die Stabilität dieses Emulsionssystems die Teilchengröße ebenso wie das Zusammenwirken der Emulgatoren von Bedeutung ist. Wesentlich ist, daß bei der Herstellung des erfindungsgemäßen kosmetischen Mittels die zum Einsatz kommenden Emulsionen dispergiert und nicht homogenisiert werden.

Die Vereinigung der beiden Emulsionen erfolgt vorzugsweise unter einem Vakuum von 0,5 torr bis 50 torr. Die Rührheschwindigkeit ist, wie dem Fachmann wohlbekannt ist, von dem verwendeten Rührwerk abhängig und muß in an sich bekannter Weise ermittelt werden.

Die Öl/Wasser-Emulsionen und die Wasser/Öl-Emulsionen können in konventioneller Weise unter Verwendung konventioneller Emulgatoren hergestellt werden (Kirk Othmer: Enzyklopedia of Chemical Technology, 3. Auflage, 1979: John Wiley & Sons; New York etc. Vol. 8, Seite 900 bis 930, und Dr. Otto-Albrecht Neumüller: Römpps Chemie Lexikon, 7. Auflage, 1973; Franck'sche Verlagshandlung Stuttgart, Seite 1009 bis 1013). Die für diese Emulsionen verwendeten Wachse, Emulgatoren und übrigen Zusätze sind die gleichen, wie man sie konventionellerweise bei emulgierten Hautpflegemitteln verwendet (Dr. Otte-Albrecht Neumüller: Römpps Chemie Lexikon, 7. Auflage, 1973; Franck'sche Verlagshandlung Stuttgart, Seite 1427 und 1428).

Eine im erfindungsgemäßen kosmetischen Mittel eingesetzte Öl/Wasser-Emulsion kann aus lipophilem Wirkstoff, Fettphase, Öl/Wasser-Emulgator, wässeriger Phase und Konservierungsmittel bestehen.

Als lipophiler Wirkstoff wird Pur-oba-Öl (= Jojoba-Öl) eingesetzt. Als ölige Phase oder als Fettphase in der Öl/Wasser-Emulsion eigen sich Kohlenwasserstoffe, wie zum Beispiel Vaseline, Paraffine oder Stearin, oder Wachse, wie zum Beispiel Bienenwachs. Geeignete Öl/Wasser-Emulgatoren sind beispielsweise Stearylalkohol, Polyoxyäthylenstearate (wie zum Beispiel MYRJ [(R)]), Komplexemulgatoren (wie zum Beispiel Amphoterin [(R)]) und Sorbitanfettsäureester (wie zum Beispiel Span [(R)]) oder Carboxyvinylpolymrisate (wie zum Beispiel Carbopol [(R)]).Die wässerige Phase kann zusätzlich noch Puffersubstanzen, wie zum Beispiel das Dinatriumsalz der Äthylendiamin-N,N,N',N'-tetraessigsäure, und Konservierungsmittel, wie Chlorquinaldol, Parabene oder Benzalkoniumchlorid enthalten.

In der Öl/Wasser-Emulsion beträgt der Anteil der inneren Emulsion vorzugsweise 30 bis 49 Gew.-%, die Teilchengröße der inneren Emulsion liegt vorzugsweise zwischen 1 $\mu$ und 100 $\mu$. Bei der Dispergierung beider Phasen wird sie nochmals zerkleinert und liegt im fertigen Produkt unterhalb von 50 $\mu$.

Eine im erfindungsgemäßen kosmetischen Mittel einsetzbare Wasser/Öl-Emulsion besteht aus Fettphase, Wasser/Öl-Emulgator und wässeriger Phase. Als ölige Phase oder Fettphase der Wasser/Öl-Emulsion können Kohlenwasserstoffe, z. B. Paraffine und Vaseline, synthetische, pflanzliche und tierische Öle bzw. Wachse (wie z. B. Olivenöl, Erdnußöl, feines Knochenöl, Mandelöl, Lanolin, Bienenwachs oder Sonnenblumenöl) eingesetzt werden, als wässerige Phase gereinigtes demineralisiertes Wasser und als Wasser/Öl-Emulgator Wollfett (= Lanolin), Fettalkohole, wie zum Beispiel Cetylalkohol, Myristylalkohol, Stearylalkohol oder Cerylalkohol, Fettsäureester, wie zum Beispiel Beinenwachs (Cera alba) oder Wachsalkoholester oder Mischester (wie zum Beispiel Dehymuls [(R)]).

In der Wasser/Öl-Emulsion beträgt der Anteil der inneren Emulsion vorzugsweise 30 bis 49 Gew.-%, die Teilchengröße der inneren Emulsion liegt vorzugsweise zwischen 1 $\mu$ und 100 $\mu$. Bei der Dispergierung beider Phasen wird sie nochmals zerkleinert und liegt im fertigen Produkt unterhalb von 50 $\mu$.

Das Mischungsverhältnis von Öl/Wasser-Emulsion und Wasser/Öl-Emulsion liegt vorzugsweise bei 20 bis 80 Gew.-% und insbesondere bei 35 % bis 65 % Öl/Wasser-Emulsion.

Das feindisperse System wird zusätzlich noch mit Duftstoffen, wie zum Beispiel diejenige der Crematest[R]-Serie, versetzt.

Das erfindungsgemäße kosmetische Mittel in Form einer Nährcreme kann beispielsweise folgende Zusammensetzung haben:

|  |  | Toleranzen |
|---|---|---|
| Pur-oba-Öl | 5 % | 5 – 10 % |
| Cera-alba (Bienenwachs) | 1 % | 1 – 5 % |
| Dehymuls [R] | 1 % | 1 – 3 % |
| Stearylalkohol | 4 % | 4 – 8 % |
| Kohlenwasserstoffe | 30 % | 30 – 50 % |
| Carbopol [R] | 1 % |  |
| MYRJ [R] | 3 % | 2 – 5 % |
| Dinatriumedetat | 1 % |  |
| Chlorquinaldol | 1 % |  |
| gereinigtes demineralisiertes Wasser | 52 % | 30 – 55 % |
| Parfümöl | 1 % |  |

Die Angaben sind in Gewichtprozent aufgeführt.

Das nachfolgende Ausführungsbeispiel dient zur Erläuterung des Herstellungsverfahrens, welches mittels allen Vorrichtungen durchgeführt werden kann, die man konventionellerweise zur Herstellung von Salben, Cremes etc. anwendet.

1. Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carboxyvinylpolymerisat [Carbopol[R], Handelsmarke der Fa. Goodrich] versetzt.

Diese Mischung wird unter kräftigem Rühren in eine Schmelze von 80,00 g Vaseline (DAB 8) - DAB 8 ist die Abkürzung für das Deutsche Arzneibuch, amtliche Ausgabe, 8. Auflage 1978 - 40,00 g Stearylalkohol, 30,00 g Polyoxyäthylenstearat [MYRJ[R], Handelsmarke der Fa. ICI America Inc. Atlas Chemicals Div.] und 50,00 g Pur-oba-Öl eingetragen. Man rührt die Mischung noch solange, bis eine Emulsion mit einer Teilchengröße von 20-70 µm entsteht.

2. Herstellung der Wasser/Öl-Emulsion.

228,00 g gereinigtes demineralisiertes Wasser werden unter kräftigem Rühren in eine Schmelze von 220,00 g Vaseline (DAB 8), 10,00 g Mischester aus Pentaerythrit, Fettalkohol, Fettsäure und Citronensäure [Dehymuls[R], Handelsmarke der Fa. Henkel KG.a.A.] und 10,00 g Cera alba eingetragen. Man rührt die Mischung noch solange, bis eine Emulsion mit einer Teilchengröße von 20-70 µm entsteht.

3. Herstellung einer Creme.

Die Wasser/Öl-Emulsion wird unter kräftigem Rühren unter einem Vakuum von 1330 Pa (10 torr) in die Öl/Wasser-Emulsion eingetragen. Man rührt noch solange, bis eine Dispersion mit einer Teilchengröße von 10 bis 50 µm entsteht, entfernt das Vakuum und setzt unter Rühren noch 2,00 g eines Deftstoffes (Crematest [R], Handelsmarke der Fa. DRAGOCO Ges. m. b. H.) zu.

**Patentansprüche**

4

1. Kosmetisches Mittel in Form einer Salbe, Paste oder Creme, das lipophilen Wirkstoff, eine Fettphase, eine wässerige Phase, Emulgatoren, Konservierungsmittel sowie Duftstoffe enthält, dadurch gekennzeichnet, daß es den lipophilen Wirkstoff, die Fettphase und wässerige Phase in Form einer feinstdispersen Mischung einer einen Öl/Wasser-Emulgator und Konservierungsmittel enthaltenden Öl/Wasser-Emulsion und einer einen Wasser/Öl-Emulgator enthaltenden Wasser/Öl-Emulsion mit einer Teilchengröße von jeweils 2 bis 50 μm enthält, wobei der Wirkstoff Pur-oba-Öl ist.

## Claims

1. Cosmetic preparation in the form of an ointment, paste or cream, comprising a lipophilic active ingredient, a fat phase, an aqueous phase, emulsifiers, preservative as well as perfumes, characterized in that it contains the lipophilic active ingredient, the fat phase and the aqueous phase in the form of a finely-dispersed mixture of an oil/water emulsion containing an oil/water emulsifier and preservative, and of a water/oil emulsion containing a water/oil emulsifier with a particle size in each case from 2 to 50 μm, wherein the active ingredient is pur-oba oil.

## Revendications

1. Produit cosmétique sous la forme d'une pommade, d'une pâte ou d'une crème, qui contient une substance active lipophile, une phase grasse, une phase aqueuse, des émulsionnants, des agents de conservation et des parfums, caractérisé en ce qu'il renferme la substance active lipophile, la phase grasse et la phase aqueuse sous la forme d'un mélange très finement dispersé d'une émulsion LH ("huile dans l'eau") contenant un émulsionnant LH et un agent de conservation et d'une émulsion HL contenant un émulsionnant HL, dont la dimension particulaire est, pour chacune d'elles, de 2 à 50 μm, la substance active étant l'huile pur-oba.